# EUROPÄISCHE PATENTSCHRIFT

(11) **EP 1 450 707 B1**
(45) Veröffentlichungstag und Bekanntmachung des Hinweises auf die Patenterteilung: **26.09.2007**
(21) Anmeldenummer: 01274864.6
(22) Anmeldetag: 07.12.2001
(51) Int. Cl.: A61B 17/70, F16F 3/10

(54) **Dämpfungselement für die Wirbelsäule**
Damping element for the spine
Élément amortisseur pour le rachis

(43) Veröffentlichungstag der Anmeldung: 01.09.2004
(73) Patentinhaber: Synthes GmbH, 4436 Oberdorf (CH)
(72) Erfinder: STUDER, Armin, CH-4513 Langendorf (CH)
(74) Vertreter: Lusuardi, Werther
(86) Internationale Anmeldenummer: PCT/CH2001/000705
(87) Internationale Veröffentlichungsnummer: WO 2003/047441

(56) Entgegenhaltungen:
- EP-A- 0 516 567
- DE-B- 1 127 671
- DE-B- 1 147 494
- FR-A- 2 774 581
- FR-A- 2 799 949
- SU-A- 313 538
- US-A- 2 322 879

## Beschreibung

Die Erfindung bezieht sich auf eine Dämpfungselement, gemäss dem Oberbegriff des Patentanspruchs 1, sowie auf eine Vorrichtung zur Stabilisierung benachbarter Wirbelkörper, gemäss dem Oberbegriff des Patentanspruchs 16.

Aus der FR-A-2 799 949 ist eine Wirbelsäulenfixations-Vorrichtung bekannt, welche aus einer Anzahl tulpenförmiger Pedikelschrauben besteht, welche statt des üblichen starren Längsträger mit einzelnen Spiralfederelementen untereinander verbunden sind. Die Länge der Spiralfedern ist zwar einstellbar, man erreicht aber damit nur eine Änderung der Federkraft zwischen zwei benachbarten Pedikelschrauben und damit zwischen zwei benachbarten Wirbelkörpern. Ob die Federelemente in einem vorgespannten Zustand zwischen den Pedikelschrauben eingesetzt werden ist aus diesem Dokument nicht ersichtlich.

Aus der EP-A-0 516 567 ist eine weitere Wirbelsäulenfixations-Vorrichtung bekannt, welche aus einer Anzahl tulpenförmiger Pedikelschrauben besteht, welche statt des üblichen starren Längsträger mit einzelnen Dämpfungselementen untereinander verbunden sind. Der Oberbegriff des Anspruchs 1 basiert aus dieser Offenbarung. Nachteilig bei dieser Vorrichtung ist, dass nur Kompressionskräfte zwischen den Pedikelschrauben aufgenommen werden können. Da die Dämpfungselemente zudem eine fixe Länge besitzen, ist vorgesehen eine grössere Anzahl solcher Dämpfungselemente mit unterschiedlicher Länge bereitzustellen, um dann ein Dämpfungselement geeigneter Länge zwischen zwei implantierte Pedikelschrauben befestigen zu können. Dies ist umständlich und bedingt eine grössere Lagerhaltung an Dämpfungselementen verschiedener Längen.

Aus der EP-B-0 669 109 ist eine weitere Wirbelsäulenfixations-Vorrichtung bekannt, welche aus einer Anzahl von Pedikelschrauben mit durchbohrtem Kopf besteht, welche statt des üblichen starren Längsträgers mit einem durch die Bohrungen der Pedikelschrauben einziehbaren elastischen Kunststoffband untereinander verbunden sind. Zwischen den einzelnen Pedikelschrauben - auf dem Kunststoffband aufgereihtsind hohlzylindrische Stützelemente, die allfällige Kompressionskräfte zwischen den Pedikelschrauben aufnehmen können. Die Nachteile dieser Vorrichtung sind vielfältig.

Erstens müssen das Kunststoffband und die Stützelemente - wie bei einer Perlenkette - in, bzw. zwischen die Bohrungen der bereits implantierten Pedikelschrauben eingefädelt werden, was für den Chirurgen umständlich und zeitraubend ist. Zweitens weist das - bis zu einem gewissen Grade elastische Kunststoffband - keine Vorspannung auf. Da die Länge der Stützkörpers auch bei dieser Vorrichtung fix ist, werden Sollbruchstellen am Stützkörper vorgeschlagen, so dass dieser intraoperativ an die effektive Distanz zwischen beiden betroffenen Pedikelschrauben vom Chirurgen zurechtgeschnitten werden kann. Dies ist für den Chirurgen umständlich und zeitraubend und dürfte in der Regel zu einem zu kurzen Stützelement führen, so dass seine Dämpfungswirkung erst mit einer gewissen Verzögerung eintritt, was natürlich unerwünscht ist.

Hier will die Erfindung Abhilfe schaffen. Der Erfindung liegt die Aufgabe zugrunde, ein kombiniertes, vorgespanntes Zug-Druck-Element zu schaffen, welches zwischen zwei Pedikelschrauben, bzw. Pedikelhaken befestigbar ist und einerseits auf Zug als Federelement mit einer gewissen Federrate und anderseits auf Druck als Dämpfungselement mit einer anderen Federrate wirkt.

Die Erfindung löst die gestellte Aufgabe mit einem Dämpfungselement, welches die Merkmale des Anspruchs 1 aufweist, sowie mit einer Vorrichtung zur Stabilisierung benachbarter Wirbelkörper, welche die Merkmale des Anspruchs 16 aufweist.

In der bevorzugten Ausführungsform des erfindungsgemässen Dämpfungselementes ist eines der Federelemente als Druckfeder angeordnet. Bei montiertem Dämpfungselement stehen die an den Enden der Federelemente anbringbaren Verbindungsteile an den Enden des als Druckfeder angeordneten Federelementes an, so dass das erste Federelement auf Zug belastet werden kann und vorspannbar ist.

Die durch die Erfindung erreichten Vorteile sind im wesentlichen die folgenden:
- einheitliches Zug/Druck-Element, dessen Länge variabel einstellbar ist;
- durch Wahl unterschiedlich langer Innenzylinder können die Dämpfungs-
- eigenschaften variiert werden.
- die bereits im vornotierten Zustand des Dämpfungselementes vorhandene Vorspannkraft ist klar definiert und kann dem Chirurgen in einer Auswahl zur Verfügung gestellt werden, welche den unterschiedlichen Körpergewichten der Patienten und den unterschiedlichen Indikationen des Eingriffs entsprechen.
- die Dämpfungselemente können nach dem Distrahieren der Wirbelkörper rasch und einfach zwischen die Pedikelschrauben eingelegt und daran fixiert werden.

In einer Ausführungsform des erfindungsgemässen Dämpfungselementes sind die Federelemente so ausgestaltet, dass sie eine konstante Federrate aufweisen. Damit ist erreichbar, dass bei Entlastung des Dämpfungselementes der Zustand der unbelasteten Federelemente wieder herstellbar ist.

In einer anderen Ausführungsform des erfindungsgemässen Dämpfungselementes weist dieses einen zur Längsachse orthogonalen, nierenförmigen Querschnitt auf. Die Vorteile einer solchen Ausführung liegen darin, dass bei der Implantation eines oder mehrerer Dämpfungselemente, beispielsweise innerhalb einer Wirbelsäulenfixation, diese mit Berücksichtigung von Wirbelfortsätzen oder anderen Implantatteilen günstiger plazierbar sind.

Weitere vorteilhafte Ausgestaltungen der Erfindung sind in den abhängigen Ansprüchen gekennzeichnet.

Die erfindungsgemässe Vorrichtung zur Stabilisierung benachbarter Wirbelkörper umfasst im wesentlichen mehrere, mit verschiedenen Befestigungsmitteln verbindbare Pedikelschrauben oder Pedikelhaken. Zwischen zwei Pedikelschrauben oder Pedikelhaken können als Befestigungsmittel beispielsweise stabförmige Längsträger, Federn oder erfindungsgemässen Dämpfungselemente eingesetzt werden.

Mindestens eine Pedikelschraube oder ein Pedikelhaken umfasst Aufnahmemittel, welche zugleich die Aufnahme von zwei parallelen longitudinalen Befestigungselementen gestattet. Auf diese Weise wird ermöglicht, dass ein als Feder wirkendes Element, ein erfindungsgemässes Dämpfungselement zur Befestigung zwischen mindestens einer mit Aufnahmemitteln versehenen Pedikelschraube oder Pedikelhaken und einer weiteren, benachbarten Pedikelschraube oder Pedikelhaken einsetzbar ist.

Pedikelschrauben oder Pedikelhaken mit Aufnahmemitteln, welche gestatten, dass zugleich zwei parallele, longitudinale Befestigungselemente mit der Pedikelschraube oder dem Pedikelhaken verbindbar sind, sind beispielsweise aus der US-A-4 653 481 HOWLAND bekannt. Die erfindungsgemässen Dämpfungselemente können analog zu den im vorangehend erwähnten Patent gezeigten Längsträgern mittels an den Verbindungsteilen parallel zur Längsachse angebrachten Stäben, beispielsweise in parallelen Kanälen an den Schraubenköpfen fixiert werden. Die durch eine solche Anordnung mögliche Verschiebbarkeit des Dämpfungselementes parallel zur Längsachse in den Kanälen gestattet, ein vor der Implantation auf eine gewünschte Federkraft vorgespanntes, erfindungsgemässes Dämpfungselement ohne weitere Manipulation am Dämpfungselement in die Aufnahmemittel an den Pedikelschrauben einzufügen. Die Längenkompensation bei verschiedenen Abständen zwischen benachbarten Pedikelschrauben oder Pedikelhaken erfolgt über die axiale Verschiebbarkeit der endständig parallel zur Längsachse angeordneten, stabförmig ausgebildeten Verbindungselemente an den erfindungsgemässen Dämpfungselemente in den ebenfalls zur Längsachse parallelen Kanälen.

Die Vorspannung des Dämpfungselementes gestattet beispielsweise die Berücksichtigung verschiedener Instabilitäten, Indikation oder des Gewichtes des Patienten. Das Dämpfungselement ist bei einer Extension der damit verbundenen Wirbelsäulenteile auf Druck belastet, während es bei einer Flexion der damit verbundenen Wirbelsäulenteile auf Zug belastet wird. Die Wahl des Federmateriales, beispielsweise ein Polymer, vorzugsweise Polycarbonaturethan (PCU) für das auf Druck belastete Federelement und ein Metall für das auch auf Zug belastete Federelement, die Wahl der geometrischen Abmessungen sowie die eingestellte Vorspannung des auch auf Zug belasteten Federelementes gestatten eine optimale Anpassung der erfindungsgemässen Vorrichtung an die biomechanischen Gegebenheiten bei einem Patienten.

Die Vorteile der erfindungsgemässen Vorrichtung sind im wesentlichen die folgenden:
- harmonischer Steifigkeitsübergang vom stabiliserten Wirbelsäulensegment zu den gesunden Wirbelsäulensegementen.
- Die Dämpfungselemente können wahlweise mit rigiden Stäben segementweise kombiniert werden.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch eine Ausführungsform des erfindungsgemässen Dämpfungselementes;
Fig. 2 eine Ansicht einer Ausführungsform der erfindungsgemässen Vorrichtung zur Stabilisierung benachbarter Wirbelkörper;
Fig. 3 eine Explosionsdarstellung einer Ausführungsform des erfindungsgemässen Dämpfungselementes;
Fig. 4 eine Ansicht einer Ausführungsform des erfindungsgemässen Dämpfungselementes;
Fig. 5 eine perspektivische Darstellung einer Ausführungsform des erfindungsgemässen Dämpfungselementes; und
Fig. 6 eine Aufsicht auf eine Ausführungsform des erfindungsgemässen Dämpfungselementes.

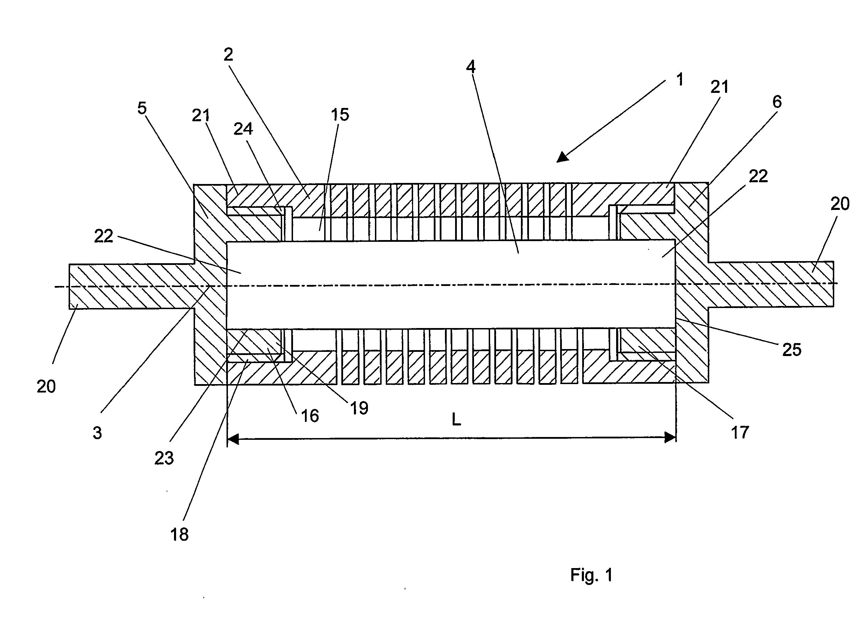

Die Fig. 1 zeigt eine Ausführungsform des erfindungsgemässen Dämpfungselementes 1 mit zwei zur Längsachse 3 konzentrisch angeordneten Federelementen 2;4. Das erste Federelement 2 ist als Schraubenfeder mit einem zentralen Hohlraum 15 ausgeführt, während das zweite Federelement 4 stabförmig ausgestaltet ist und im Hohlraum 15 angeordnet ist. Die endständigen Verbindungsteile 5;6 sind ebenfalls zur Längsachse 3 koaxial angeordnet und weisen gegen die Federelemente 2;4 gerichtet, je ein zur Längsachse 3 koaxiales Gewindestück 16;17 mit einem Aussengewinde 18 auf. Das erste Federelement 2 ist an seinen axialen Enden 21 mit im Hohlraum 15 angebrachten Innengewinden 24 versehen, welche zu den Aussengewinden 18 komplementär ausgestaltet sind, so dass die Gewindestücke der Verbindungsteile 5;6 in das erste Federelement 2 einschraubbar sind. Ferner umfasst jedes Verbindungsteil 5;6 eine zur Längsachse 3 koaxial angeordnete und am inneren Ende 19 des Verbindungsteils 5;6 offene Vertiefung 23, so dass das stabförmig ausgebildete zweite Federelement 4 an seinen axialen Enden 22 in den Vertiefungen 23 aufnehmbar ist. Ferner sind die Verbindungsteile 5;6 an ihren äusseren Ende 20 koaxial stabförmig ausgebildet. Bei montiertem Dämpfungselement 1 liegen die Enden 22 der zweiten Feder 4 an den zur Längsachse 3 orthogonalen Stirnflächen 25 der Vertiefungen 23 auf, so dass die Verbindungsteile 5;6 zwischen diesen Stirnflächen 25 einen Abstand L aufweisen. Dieser Abstand L sowie die Länge des nicht deformierten ersten Federelementes 2 sind so bemessen, dass beim Einschrauben der Gewindestücke 16;17 in die Innengewinde 24 das erste Federelement 2 axial um eine gewünschte Länge gedehnt wird, wodurch das Dämpfungselement 1 eine Vorspannung erhält.

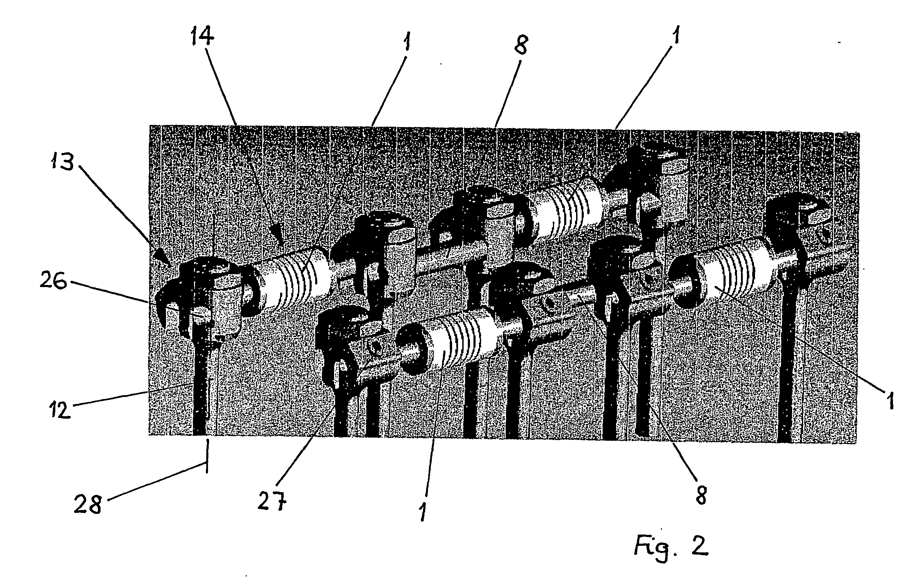

In Fig. 2 ist eine Ausführungsform der erfindungsgemässen Vorrichtung am Beispiel einer Vorrichtung zur Stabilisierung benachbarter Wirbelkörper (nicht gezeichnet) dargestellt. Mehrere Pedikelschrauben oder -haken 12 sind so an den Pedikeln der zu verbindenden Wirbelkörper befestigt, dass ihre Zentralachsen 28 quer zur Wirbelsäulenlängsachse angeordnet sind. Die Aufnahmemittel 13 an den Pedikelschrauben oder -haken 12 sind zu den Zentralachsen 28 senkrecht angeordnet und als Kanäle 26 ausgebildet. In diesen Kanälen 26 sind die stabförmigen, äusseren Enden 20 der Verbindungsteile 5;6 (Fig. 1) einführbar, so dass die Dämpfungselemente 1 in den Kanälen 26 axial verschiebbar sind, bevor sie mittels Schrauben 27 relativ zu den Pedikelschrauben oder -haken 12 fixiert werden. Die Aufnahmemittel 13 an den Pedikelschrauben oder -haken 12 umfassen je zwei parallele Kanäle 26, so dass an einer Pedikelschraube oder -haken 12 neben einem Dämpfungselement 1 beispielsweise ein stabförmiges Befestigungselement 7 fixierbar ist.

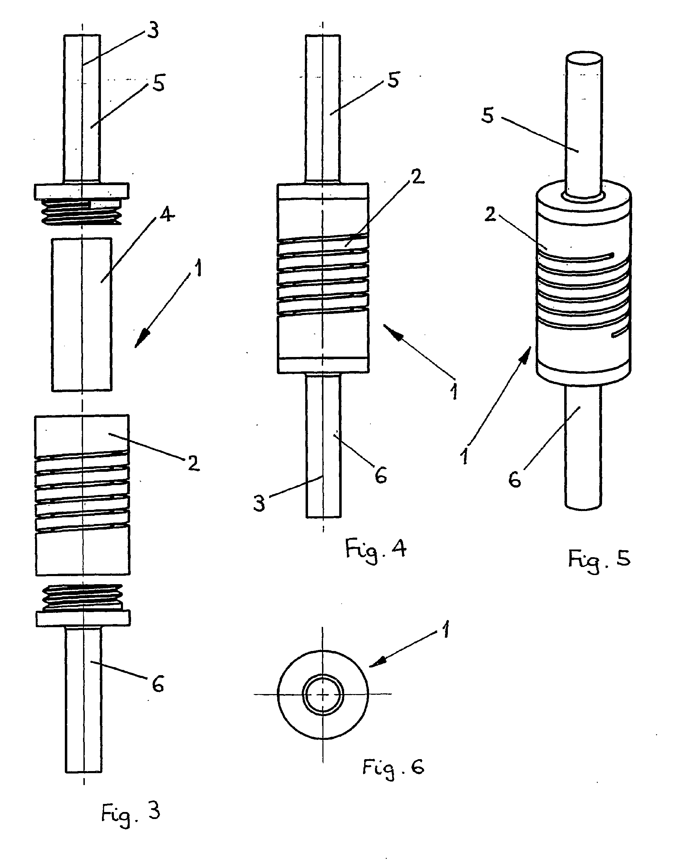

Fig. 3 zeigt eine Ausführungsform des erfindungsgemässen Dämpfungselementes 1 mit einem als Schraubenfeder ausgestalteten, ersten Federelement 2, einem stabförmig ausgebildeten, zweiten Federelement 4 und zwei zur Längsachse 3 koaxial angeordneten Verbindungsteilen 5;6.

Fig. 4 und 5 zeigen eine Ausführungsform des erfindungsgemässen Dämpfungselementes 1 mit einem als Schraubenfeder ausgestalteten ersten Federelement 2 und zwei zur Längsachse 3 koaxial mit dem ersten Federelement 2 verbundenen Verbindungsteilen 5;6.

In Fig. 6 ist eine Ausführungsform des erfindungsgemässen Dämpfungselementes 1 dargestellt, welches einen zur Längsachse 3 orthogonalen, kreisförmigen Querschnitt aufweist. Andere Querschnittsformen, beispielsweise ovale oder elliptische Querschnitte, welche die Implantation des Dämpfungselementes 1 begünstigen sind ebenfalls möglich.

## Patentansprüche

1. Dämpfungselement (1), welches zwischen zwei Pedikelschrauben, bzw. Pedikelhaken befestigbar ist, mit
A) zwei zu einer Längsachse (3) koaxialen oder parallelen Federelemente (2;4) und axial endständig zwei Verbindungsteilen (5;6), welche mit den Federelementen (2;4) so zusammenfügbar sind, dass mindestens eines der Federelemente (2;4) mit den Verbindungsteilen (5;6) verbunden ist; wobei
B) das erste Federelement (2) eine Federrate F aufweist;
C) das zweite Federelement (4) eine Federrate f aufweist; und
D) die Federraten F und f voneinander verschieden sind,
**dadurch gekennzeichnet, dass**
E) ein Federelement (2) eine Federwendel aufweist und dass
F) sich die beiden Federraten F;f mindestens um den Faktor 2 unterscheiden.

2. Dämpfungselement (1) nach Anspruch 1, **dadurch gekennzeichnet, dass** die Federelemente (2;4) zur Längsachse (3) konzentrisch angeordnet sind.

3. Dämpfungselement (1) nach Anspruch 1 oder 2, **dadurch gekennzeichnet, dass** mindestens eines der Federelemente (2;4) vorgespannt ist.

4. Dämpfungselement (1) nach einem der Ansprüche 1 bis 3, **dadurch gekennzeichnet, dass** die Federelemente (2;4) konstante Federraten F;f aufweisen.

5. Dämpfungselement (1) nach einem der Ansprüche 1 bis 4, **dadurch gekennzeichnet, dass** es einen zur Längsachse (3) orthogonalen, nierenförmigen Querschnitt aufweist.

6. Dämpfungselement (1) nach einem der Ansprüche 1 bis 5, **dadurch gekennzeichnet, dass** ein Federelement (4) als Druckfeder angeordnet ist.

7. Dämpfungselement (1) nach einem der Ansprüche 1 bis 6, **dadurch gekennzeichnet, dass** ein Federelement (2;4) mit einer mehrgängigen Federwendel ausgestattet ist.

8. Dämpfungselement (1) nach Anspruch 6 oder 7, **dadurch gekennzeichnet, dass** das als Druckfeder angeordnete Federelement (4) aus einem Polymer, vorzugsweise aus Polycarbonaturethan (PCU) besteht.

9. Dämpfungselement (1) nach einem der Ansprüche 1 bis 8, **dadurch gekennzeichnet, dass** das zweite Federelement (4) mit einem der Verbindungsteile (5;6) einstückig ausgebildet ist.

10. Dämpfungselement (1) nach einem der Ansprüche 1 bis 9, **dadurch gekennzeichnet, dass** das zweite Federelement (4) im Inneren des ersten Federelementes (2) angeordnet ist.

11. Dämpfungselement (1) nach einem der Ansprüche 1 bis 10, **dadurch gekennzeichnet, dass** sich die beiden Federkonstanten F;f mindestens um den Faktor 5 unterscheiden.

12. Dämpfungselement (1) nach Anspruch 11, **dadurch gekennzeichnet, dass** sich die beiden Federkonstanten F;f um einen Faktor zwischen 10 und 100 unterscheiden.

13. Dämpfungselement (1) nach einem der Ansprüche 1 bis 12, **dadurch gekennzeichnet, dass** die Federrate f des zweiten Federelementes (4) zwischen 100 N/mm und 5000 N/mm beträgt.

14. Dämpfungselement (1) nach Anspruch 13, **dadurch gekennzeichnet, dass** die Federrate f des zweiten Federelementes (4) zwischen 200 N/mm und 2000 N/mm beträgt.

15. Dämpfungselement (1) nach einem der Ansprüche 1 bis 14, **dadurch gekennzeichnet, dass** es ein Dämpfungselement (1) in einer Vorrichtung zur Stabilisierung benachbarter Wirbelkörper ist.

16. Vorrichtung zur Stabilisierung benachbarter Wirbelkörper mit einem Dämpfungselement (1) nach einem der Ansprüche 1 bis 15 und
A) N Pedikelschrauben oder Pedikelhaken (12), wobei N ≥ 3 ist; und
B) wovon mindestens eine Pedikelschraube oder ein Pedikelhaken (12) Aufnahmemittel (13) umfasst, welche zugleich die Aufnahme von zwei parallelen longitudinalen Befestigungselementen (7) gestattet,
**dadurch gekennzeichnet dass**,
ein Dämpfungselement (1) zur Befestigung zwischen der mindestes einen mit Aufnahmemitteln (13) versehenen Pedikelschraube oder Pedikelhaken (12) und einer weiteren, benachbarten Pedikelschraube oder Pedikelhaken (12) vorgesehen ist.

## Claims

1. A damping element (1), which is fixable between two pedicle screw, respectively pedicle hooks, comprising
(a) two spring elements (2 , 4) coaxial with or parallel to a longitudinal axis (3), and two axially end-side connectors (5, 6) which can be linked to the spring elements (2, 4) in a manner that at least one of the spring elements (2, 4) is connected to the connectors (6, 7), whereby
(b) the first spring element (2) exhibits a spring rate F,
(c) the second spring rate (4) exhibits a spring rate f, and
(d) the spring rates F and f are different from each other
**characterized in that**
(e) one spring element (2) comprises a spring coil and that
(f) the two spring rates F;f differ at least by a factor 2.

2. Damping element (1) as claimed in claim 1, **characterized in that** the spring elements (2, 4) are configured concentrically with the longitudinal axis (3).

3. Damping element (1) as claimed in either of claims 1 and 2, **characterized in that** at least one spring element (2, 4) is prestressed.

4. Damping element (1) as claimed in one of claims 1 through 3, **characterized in that** the spring elements (2, 4) exhibit constant spring rates F, f.

5. Damping element (1) as claimed in one of claims 1 through 4, **characterized in that** its cross-section orthogonal to the longitudinal axis (3) is reniform.

6. Damping element (1) as claimed in one of claims 1 through 5, **characterized in that** one spring element (4) is a compression spring.

7. Damping element (1) as claimed in one of claims 1 through 6, **characterized in that** one spring element (2, 4) is fitted with a multiple spring coil.

8. Damping element (1) as claimed in either of claims 6 and 7, **characterized in that** the spring element (4) in the form of a compression spring is made of a polymer, preferably a polycarbonate urethane (PCU).

9. Damping element (1) as claimed in one of claims 1 through 8, **characterized in that** the second spring element (4) is integral with one of the connectors (5, 6).

10. Damping element (1) as claimed in one of claims 1 through 9, **characterized in that** the second spring element (4) is configured inside the first spring element (2).

11. Damping element (1) as claimed in one of claims 1 through 10, **characterized in that** the two spring constants F, f differ at least by the factor 5.

12. Damping element (1) as claimed in claim 11, **characterized in that** the two spring constants differ F, f by a factor between 10 and 100.

13. Damping element (1) as claimed in one of claims 1 through 12, **characterized in that** the spring rate F of the second spring element (4) is between 100 N/mm and 5,000 N/mm.

14. Damping element (1) as claimed in claim 13, **characterized in that** the spring rate f of the second spring element (4) is between 200 N/mm and 2,000 N/mm.

15. Damping element (1) as claimed in one of claims 1 through 14, **characterized in that** it is a damping element (1) in a device for stabilizing adjacent vertebras.

16. Device for stabilizing adjacent vertebras with a damping element (1) according to one of the claims 1 through 15 and
(a) N pedicle screws or pedicle hooks (12), where N ≥ 3, and
(b) at least one pedicle screw or one pedicle hook (12) comprises receiving means (13) simultaneously allowing receiving two parallel longitudinal affixation means (7)
**characterized in that**
a damping element (1) is used for affixation between the at least one pedicle screw or pedicle hook (12) fitted with receiving means (13) and a further adjacent pedicle screw or pedicle hook (12).

## Revendications

1. Élément amortisseur (1), qui peut être fixé entre deux vis pédiculaires ou crochets pédiculaires, comprenant
A) deux éléments à ressort (2 ; 4) coaxiaux ou parallèles à un axe longitudinal (3) et, positionnés sur l'axe et aux extrémités, deux éléments de liaison (5 ; 6), qui peuvent être assemblés avec les éléments à ressort (2 ; 4) de façon à ce qu'au moins un des éléments à ressort (2 ; 4) soit relié aux éléments de liaison (5 ; 6) ; où
B) le premier élément à ressort (2) présente une constante de rappel F ;
C) le deuxième élément à ressort (4) présente une constante de rappel f; et
D) les constantes de rappel F et f sont différentes l'une de l'autre,
**caractérisé en ce que**
E) un élément à ressort (2) présente un ressort hélicoïdal, et **en ce que**
F) les deux constantes de rappel F ; f diffèrent au moins d'un facteur 2.

2. Élément amortisseur (1) selon la revendication 1, **caractérisé en ce que** les éléments à ressort (2 ; 4) sont disposés concentriquement à l'axe longitudinal (3).

3. Élément amortisseur (1) selon la revendication 1 ou 2, **caractérisé en ce qu'**au moins un des éléments à ressort (2 ; 4) est précontraint.

4. Élément amortisseur (1) selon l'une quelconque des revendications 1 à 3, **caractérisé en ce que** les éléments à ressort (2 ; 4) présentent des constantes de rappel F ; f invariables.

5. Élément amortisseur (1) selon l'une quelconque des revendications 1 à 4, **caractérisé en ce qu'**il présente une section réniforme orthogonale à l'axe longitudinal (3).

6. Élément amortisseur (1) selon l'une quelconque des revendications 1 à 5, **caractérisé en ce qu'**un élément à ressort (4) est prévu en tant que ressort de compression.

7. Élément amortisseur (1) selon l'une quelconque des revendications 1 à 6, **caractérisé en ce qu'**un élément à ressort (2 ; 4) est pourvu d'un ressort hélicoïdal à spires multiples.

8. Élément amortisseur (1) selon la revendication 6 ou 7, **caractérisé en ce que** l'élément à ressort (4) prévu en tant que ressort de compression est fait d'un polymère, de préférence de polycarbonate-uréthanne (PCU).

9. Élément amortisseur (1) selon l'une quelconque des revendications 1 à 8, **caractérisé en ce que** le deuxième élément à ressort (4) est formé d'une seule pièce avec l'un des éléments de liaison (5 ; 6).

10. Élément amortisseur (1) selon l'une quelconque des revendications 1 à 9, **caractérisé en ce que** le deuxième élément à ressort (4) est disposé à l'intérieur du premier élément à ressort (2).

11. Élément amortisseur (1) selon l'une quelconque des revendications 1 à 10, **caractérisé en ce que** les deux constantes de rappel F ; f diffèrent au moins d'un facteur 5.

12. Élément amortisseur (1) selon la revendication 11, **caractérisé en ce que** les deux constantes de rappel F ; f diffèrent d'un facteur compris entre 10 et 100.

13. Élément amortisseur (1) selon l'une quelconque des revendications 1 à 12, **caractérisé en ce que** la constante de rappel f du deuxième élément à ressort (4) est comprise entre 100 N/mm et 5 000 N/mm.

14. Élément amortisseur (1) selon la revendication 13, **caractérisé en ce que** la constante de rappel f du deuxième élément à ressort (4) est comprise entre 200 N/mm et 2 000 N/mm.

15. Élément amortisseur (1) selon l'une quelconque des revendications 1 à 14, **caractérisé en ce qu'**il s'agit d'un élément amortisseur (1) qui fait partie d'un dispositif de stabilisation de corps vertébraux adjacents.

16. Dispositif de stabilisation de corps vertébraux adjacents comprenant un élément amortisseur (1) selon l'une quelconque des revendications 1 à 15, et
A) N vis pédiculaires ou crochets pédiculaires (12), où N ≥ 3 ; et
B) dont au moins une vis pédiculaire ou un crochet pédiculaire (12) comprend des éléments de réception (13) qui permettent de recevoir en même temps deux éléments de fixation longitudinaux parallèles (7),
**caractérisé en ce que**
un élément amortisseur (1) est prévu pour une fixation entre la au moins une vis pédiculaire ou le au moins un crochet pédiculaire (12) pourvu de moyens de réception (13) et l'autre vis pédiculaire ou crochet pédiculaire adjacent (12).
